# EUROPEAN PATENT APPLICATION

(11) **EP 4 603 030 A1**
(43) Date of publication of application: **20.08.2025**
(21) Application number: 24158488.7
(22) Date of filing: 19.02.2024
(51) Int. Cl.: A61B 17/00

(54) **COLLAPSIBLE MEDICAL DEVICE**

(71) Applicant: Zahid, Amin, Winter Park, FL 32789 (US)
(72) Inventor: Zahid, Amin, Winter Park, FL 32789 (US)
(74) Representative: DTS Patent- und Rechtsanwälte PartmbB

(57) **Abstract**

The present invention provides collapsible medical device (100), preferably an occluder device, comprising a fabric formed of braided strands (10), the device (100) having a collapsed configuration for delivery through at least a lumen (L) in a subject, preferably a human patient, and has a generally dumbbell-shaped expanded configuration with two expanded diameter portions (12, 14) spaced apart by a waist portion (16) formed between the two expanded diameter portions (12, 14).

## Description

The present invention belongs to the technical field of collapsible medical devices in particular for treatment of a cardiac defect or hole, preferably a septal defect or hole, e.g. a septal defect or hole caused by a congenital heart disease.

The medical device can be an occluder device, in particular a heart occluder device for closing a cardiac defect or hole, preferably a septal defect or hole.

In particular, cardiac defects or holes, such as septal defects or holes, represent unwanted openings formed in a heart wall.

Congenital heart diseases are among the most frequent defects children are born with.

The most common congenital heart defects are atrial septal defects (ASD), ventricular septal defects (VSD), patent foramen ovale (PFO), and patent ductus arteriosus (PDA).

Defects of this kind may affect different regions of the heart, creating abnormal communication between two chambers that affects physiology of blood circulation.

Some of these defects are treated and repaired through minimally invasive, interventional techniques.

Alternatively, or in addition, medical devices, in particular occluder devices, may be implanted to mechanically close the cardiac defect or hole.

Medical devices, in particular occluder devices, of this kind are known in the art.

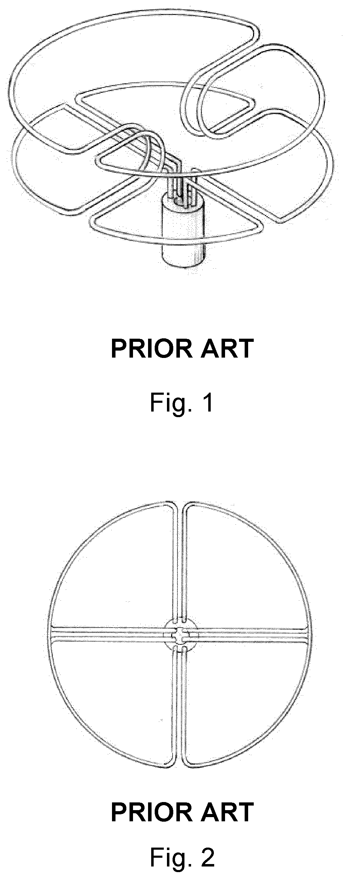

U.S. Patent 9,119,607 discloses a heart occlusion device having a self-centering mechanism, an example of which is shown in **Figs. 1-2**.

This known device comprises two separate, uniquely-shaped wires, each forming shapes that mirror the respective wire's shapes. In particular, each wire forms half-discs or quarter-discs that together form a distal disc and a proximal disc. The distal disc and proximal disc are separated by a self-centering waist.

In another configuration, this known device includes four separate wires, each mirroring its neighboring wire and forming a proximal and a distal quarter-disc. Here, the quarter-discs of each wire together form proximal and distal discs.

At least the proximal disc is attached to a hub comprising a screw mechanism. The discs further include coverings forming a sealant to occlude an aperture in a tissue, e.g. cardiac tissue. The wires forming the discs have shape-memory capability such that they can be collapsed and distorted in a catheter during delivery, but resume and maintain their intended shape after delivery.

European Patent No. 0808138 discloses a medical device, in particular an intravascular occlusion device, formed of a metal fabric. The device is capable of switching between an expanded configuration and a collapsed configuration. In particular, the medical device is collapsed for deployment through a catheter. Then, upon exiting the distal end of the catheter in a patient's channel, will resiliently substantially return to its expanded configuration.

This known medical device has a generally tubular middle portion and a pair of disc-shaped expanded diameter portions, with one expanded diameter portion positioned at either end of the middle portion.

Although effective, these known medical devices still have several drawbacks.

Most of these issues are inherent to the self-centering natural of the device, and in particular pertain to the structure of the waist portion.

A significant limitation of these known medical devices is that the two discs are completely dependent on each other.

Thus, in the common case where one of the discs is fixed, the other disc will be fixed by default because it is connected with the same wires via the waist.

Accordingly, if the edge of the medical device, e.g. an occluder device, is in contact with a tissue, e.g. a cardiac tissue, and the device remains stuck in one orientation/configuration, a sawing effect may occur, which may significantly damage or even perforate the tissue.

Also, difficulties have been reported during device deployment, especially in ASD and VSD, mostly because the device discs and the septal angle tend to mismatch, despite improvement in attaching mechanisms (like change of angle of the clamp or discs or the connecting cable)

Other complications are related to erosion and undue radial force exhibited by the waist portion of the device, which may inter alia increase arrhythmias.

A device that conforms to differences in septal tissue and morphology without increasing tension on the septum would therefore be needed.

In the light of the above, it is an object of the present invention to provide a collapsible medical device, preferably an occluder device, which can be easily deployed and reliably maintained in place after deployment without the risk of tissue damage or perforation, and/or device prolapse.

The above-specified object is achieved by the provision of a collapsible medical device as defined in claim 1.

According to the invention, a collapsible medical device is provided, comprising a fabric formed of braided strands, the device having a collapsed configuration for delivery through at least a lumen in a subject, preferably a human patient, and has a generally dumbbell-shaped expanded configuration with two expanded diameter portions spaced apart by a waist portion formed between the two expanded diameter portions.

The present invention provides a collapsible medical device.

In particular, said collapsible medical device can be an occluder device.

The medical device comprises a fabric formed of braided strands.

The medical device has a collapsed configuration, in particular for delivery of the medical device through at least a lumen of a subject, e.g. the vena cava, by using a catheter.

The medical device also has a generally dumbbell-shaped expanded configuration.

Here, two expanded diameter portions of the medical device are spaced apart by a waist portion.

In particular, the waist portion is formed between the two expanded diameter portions. These expanded diameter portions are also each called discs (as they have/can have e.g. a disc-like form).

The discs can be chosen according to the needs of the defect or hole to be closed. The can be equal in size. Or, they can be chosen with different diameter, depending on the place to be deployed and implanted.

The two expanded diameter portions comprise a first expanded diameter portion and a second expanded diameter portion.

The first expanded diameter portion is oriented toward the left side after implantation. Thereby, the left side is the side, which will be placed after surgical placement in the left atrium or left ventricle/left side of the heart.

The second expanded diameter portion is oriented toward the right side after implantation. Thereby, the right side is the side, which will be placed after surgical placement in the right atrium or right ventricle/right side of the heart.

The invention is based on the basic idea that, by the provision of a medical device, preferably an occluder device, so configured, the self-centering properties of the device can be preserved, while the two expanded diameter portions are independent from the waist portion. Accordingly, the medical device can be more easily deployed and maintained in place after implantation, preventing the occurrence of damage or perforation, and/or device prolapse.

Advantageously, the waist portion can be configured and arranged such that the expanded diameter portions are semi-independent in their movement to each other.

In other words, in an example embodiment of the device, which is fully deployable or deployed to or in a patient, the waist portion allows that the expanded diameter portions are semi-independent in their movement to each other.

The semi-independent movement of the expanded diameter portions, especially during endothelialization (occurring in the first months after implantation), allows further preventing the risk of tissue trauma.

Advantageously, the waist portion can be configured and arranged to at least partially control the movement of the expandable diameter portions.

In other words, in an example embodiment of the device, which is fully deployable or deployed to or in a patient, the waist portion is controlling at least partially the movement of the expandable diameter portions. Accordingly, self-centering properties of the medical device can be maintained, at the same time allowing for an easier deployment, causing less tissue friction and/or trauma.

Also, due to its improved adaptability to the surrounding anatomy, the medical device can be reliably used for treatment of every type of cardiac defect or hole, e.g. caused by ASD, VSD, PFO, and/or PDA.

The waist portion may be configured to connect the expandable diameter portions to one another.

In other words, in an example embodiment of the device, which is fully deployable or deployed to or in a patient, the waist portion is connecting the expandable diameter portions to one another.

This allows the waist portion to directly control the movement, preferably semi-independent movement, of the expandable diameter portions.

The waist portion could be made of a fabric, like Dacron ^{®} (= medical grade fabric of polyethyleneterephthalate (PET), provided by Dupont), polytetrafluorethylene (PTFE), polyamide (PA), or any other suitable material.

The waist portion can also be formed mainly by several rings.

The waist portion may be configured and arranged to be placed in a cardiac defect or hole.

Preferably, said cardiac defect or hole is a septal defect or hole caused by a congenital heart disease such as ASD, VSD, PFO, and/or PDA.

Preferably, the waist portion may be configured and arranged to be at least partially self-centering in said cardiac defect or hole, e.g. a septal defect or hole.

This allows obtaining an enhanced adaption to a surrounding anatomy.

Partial-self centering is convenient when the medical device is used for treatment of a cardiac defect or hole caused by muscular VSD (MVSD).

Alternatively, the waist portion can be fully self-centering.

The two expanded diameter portions may be configured and arranged to be slidable relatively to each other, controlled in their relative movement by the waist portion.

This allows for easier deployment at the target site, further reducing the risk of tissue damage and/or unwanted device displacement.

The two expanded diameter portions may be configured and arranged to be at least partly rotatable relatively to each other, controlled in their relative movement by the waist portion.

In other words, in an example embodiment of the device, which is fully deployable or deployed to or in a patient, the two expanded diameter portions are at least partly rotatable relatively to each other, controlled in their relative movement by the waist portion.For example, the expanded diameter portions may be partly rotatable relatively to each other.

Alternatively, the expanded diameter portions may be fully rotatable relatively to each other.

This allows avoiding the occurrence of a sawing effect to a surrounding tissue at the implantation site, thereby preventing tissue trauma. Unwanted erosion of the heart tissue and also a perforation of the heart can be prevented.

The two expanded diameter portions may be configured and arranged to adjust their distance relatively to each other, controlled in their relative movement by the waist portion.

This allows for better adaption to the surrounding anatomy at the implantation site.

The braided strands forming the fabric can be made of metal.

For instance, said metal may include Nitinol or Steel.

Alternatively, the braided strands forming the fabric can be made of a polymeric material.

For instance, said polymeric material can be Polyester. Further, it can be any fabric, like Dacron ^{®} (= medical grade fabric of polyethyleneterephthalate (PET), provided by Dupont), polytetrafluorethylene (PTFE), polyamide (PA), or any other suitable material.

Optionally, said polymeric material, e.g. Polyester, can be provided with a metal covering.

For instance, said metal covering may comprise Nitinol or Steel.

Each of the two expanded diameter portions may include a respective waist element.

In particular, the waist elements of the expanded diameter portions are made of the same braided strands forming the expanded diameter portions.

The waist elements of the two expanded diameter portions form the waist portion, when joined to one another.

Each of the waist elements is connected to the respective expanded diameter portion at one or more attachment points.

This configuration allows further ensuring the self-centering characteristics of the medical device.

One or more connection elements may be provided to connect the waist elements of the expanded diameter portions to one another to form the waist portion.

Preferably, said waist elements are connected to one another through a plurality of connection elements.

This allows achieving a more reliable connection.

Preferably, the one or more connection elements are provided on the waist element of the first expanded diameter portion.

In particular, the one or more connection elements may be free to rotate and may be loosely attached to the waist element of the first expanded diameter portion.

The one or more connection elements may be circular, partially rectangular, oval, triangular, or quadratic in cross-section.

Oval-shaped connection elements are particularly convenient in case the medical device is used in the treatment of PFO, as it renders said one or more connection elements more slidable in a tunnel-type PFO.

Preferably, said one or more connection elements have rounded edges.

This allows facilitating implantation and/or further preventing the risk of damages to a surrounding tissue during and/or after deployment.

Alternatively, said one or more connection elements may be string-shaped.

Alternatively, said one or more connection elements may be substantially in the shape of an elongated "8".

The optimal number of connection elements varies based upon the dimensions (e.g. the diameter) of the waist portion.

For instance, a waist portion having a larger size may require an increased number of connection elements in order to ensure a reliable connection.

The one or more connection elements may be arranged such that the two expanded diameter portions are spaced apart by a distance ranging from 6 mm to 9 mm.

The latter configuration is particularly convenient in case the medical device is used for treatment of cardiac defects or holes caused by MVSD.

The first expanded diameter portion may have a larger diameter than the second expanded diameter portion. This can help to adapt better to the anatomy of the patient. Further, the discs may stay this way (depending on the anatomy of the patient) independent in their movement.

Alternatively, the two expanded diameter portions may have the same diameter.

In one aspect, at least the second expanded diameter portion can be 1 mm to 3 mm larger than the waist.

This configuration is particularly convenient in case the medical device is used for treatment of cardiac defects or holes caused by PDA. In one aspect, the expanded diameter portion may be configured and arranged to entirely cover a cardiac defect or hole, preferably a septal defect or hole, while the first expanded diameter portion may be configured and arranged to act as an anchor element, securing the medical device to the surrounding tissue of the lumen after implantation.

This configuration is particularly convenient in case the medical device is used for treatment of cardiac defects or holes caused by VSD.

In one aspect, the first expanded diameter portion may have a concave shape, in particular a mildly-concave shape.

This allows achieving a better adaption to the anatomy of a surrounding body cavity after implantation.

Preferably, said body cavity can be the inner aortic wall.

In one aspect, the two expanded diameter portions may have a double-layered structure.

This enhances the overall reliability of the device and ensures complete closure of the cardiac defect or hole.

In one aspect, the waist portion may have a circular shape.

Here, in principle, it is preferable that the waist portion has a diameter that is generally equal to the size of the cardiac defect or hole to be treated.

For this purpose, the waist portion may have a diameter ranging between 4 mm and 48 mm.

Alternatively, the waist portion may have an oval shape.

Alternatively, the waist portion may have a substantially lunar shape.

The latter configuration allows for better fitting with lunar-shaped cardiac defects or holes, which are particularly common in case of PFO.

This also ensures an improved coverage of the ventricular septum in case of perimembranous ventricular septal defect (PMVSD).

In one aspect, the waist portion may include a mesh made of a polymeric material.

Preferably, the waist portion includes a double mesh made of a polymeric material.

This allows ensuring an effective seal for the treated cardiac defect or hole.

Preferably, said polymeric material is Polyester (forming a so-called Dacron^{®} mesh).

In one aspect, the waist portion may have a variable length.

This allows obtaining improved adaption to the surrounding anatomy.

In one aspect, the waist portion may have an overall length from 3 mm to 40 mm.

In principle, optimal dimensions for the waist portion are defined based on the size of the cardiac defect or hole to be treated.

As mentioned, the collapsible medical device of the invention is preferably an occluder device.

Said occluder device may be in particular adapted for use in a subject, preferably a human patient, affected by a congenital heart disease such as ADS, VSD, PFO and/or PDA.

Further advantages of the present invention shall now be disclosed in connection with the drawings, where:
- **Figs. 1-2**: show an exemplary medical device, in particular a heart occluder device, according to the prior art;
- **Fig. 3**: shows a collapsible medical device according to an embodiment of the invention, here an occluder device, in a relaxed position before deployment, where first and second expanded diameter portions overlap each other;
- **Fig. 4**: is a view similar to that of **Fig. 1**, but in partial cross-section. A waist portion separates the expanded diameter portions from each other. The expanded diameter portions comprise respective waist elements. The waist elements are joined together through a plurality of connection elements, here in the shape of rings, thereby forming the waist portion;
- **Fig. 5**: shows the collapsible medical device of **Fig. 1** while being delivered by using a catheter. For instance, the medical device can be delivered through the vena cava of a patient though a catheter;
- **Fig. 6**: shows the collapsible medical device of **Fig. 1** in a deployed position, placed in a cardiac defect or hole, preferably a septal defect or hole. Here, the medical device generally has dumbbell-shaped expanded configuration, where the first and second expanded diameter portions are spaced apart from one another;
- **Fig. 7**: shows a perspective view of the first expanded diameter portion from below. A plurality of ring-shaped connection elements is arranged around the waist element of the first expanded diameter portion;
- **Fig. 8**: shows a perspective view of the second expanded diameter portion from below;
- **Fig. 9**: shows a detail of the waist portion of the medical device according to the invention, e.g. the occluder device of **Fig. 1****.** The waist portion is formed by the waist portions of the expanded diameter portions, joined to one another through a plurality of connection elements, here ring-shaped connection elements;
- **Fig. 10**: shows one of the waist elements forming the waist portion of the collapsible medical device of the invention, e.g. the occluder device illustrated in **Fig. 1****.** Here, the waist element includes a plurality of wire loops;
- **Fig. 11**: shows a detail of one of the wire loops if the waist element of **Fig. 10****.** here, the wire loop has a substantially oval shape.

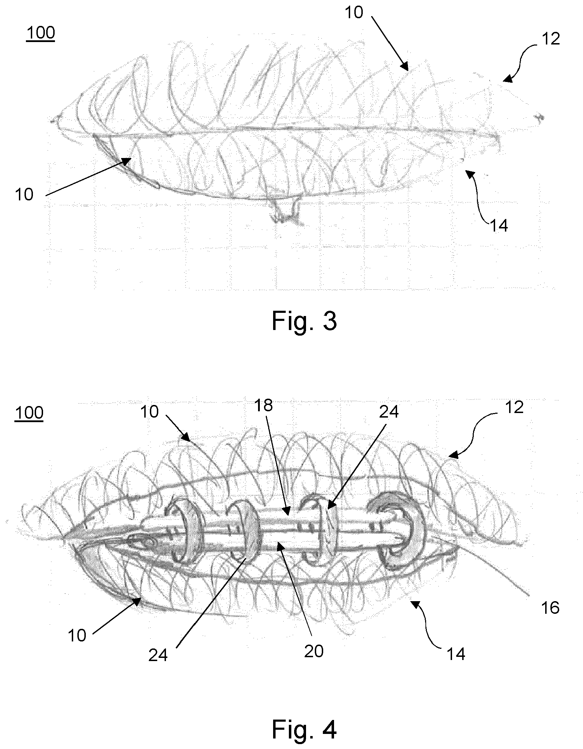

**Fig. 3** shows a collapsible medical device 100 according to an embodiment of the invention, in a relaxed position prior to deployment.

**Fig. 4** provides a view that is similar to that of **Fig. 3**, but in partial cross-section, so that otherwise-hidden components of the medical device 100 are rendered visible.

In the present embodiment, the medical device 100 is an occluder device 100, in particular adapted for use in a subject, preferably a human patient, affected by a congenital heart disease such as atrial septal defects (ASD), ventricular septal defects (VSD), patent foramen ovale (PFO), and/or patent ductus arteriosus (PDA).

The medical device 100 comprises a fabric formed of braided strands 10.

The medical device 100 comprises two expanded diameter portions 12, 14.

As shown in **Fig. 4**, the two expanded diameter portions 12, 14 are separated by a waist portion 16, formed between the two expanded diameter portions 12, 14.

The two expanded diameter portions 12, 14 can also be called discs 12, 14.

The waist portion 16 will be described in greater detail in the following.

The medical device 100, here an occluder device 100, has a collapsed configuration, for delivery through a lumen L, e.g. the vena cava of a patient.

In this configuration, the two expanded diameter portions 12, 14 overlap each other (**Figs. 3-4**).

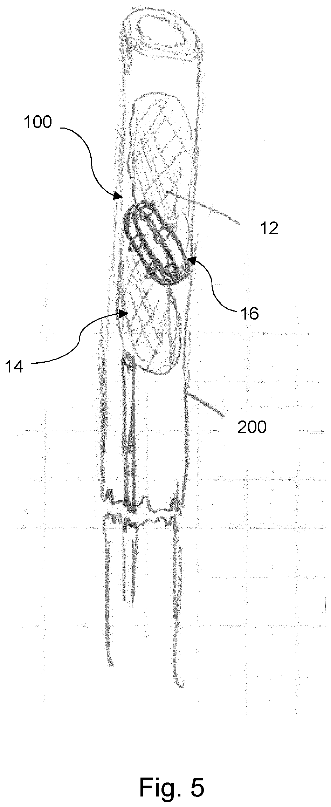

The medical device 100 may be deployed through minimally-invasive surgical techniques by using a catheter 200, as schematically illustrated in **Fig. 5****.**

Surgical techniques of this kind are well-known in the art.

The medical device 100 further has a generally dumbbell-shaped expanded configuration, after deployment at a target site.

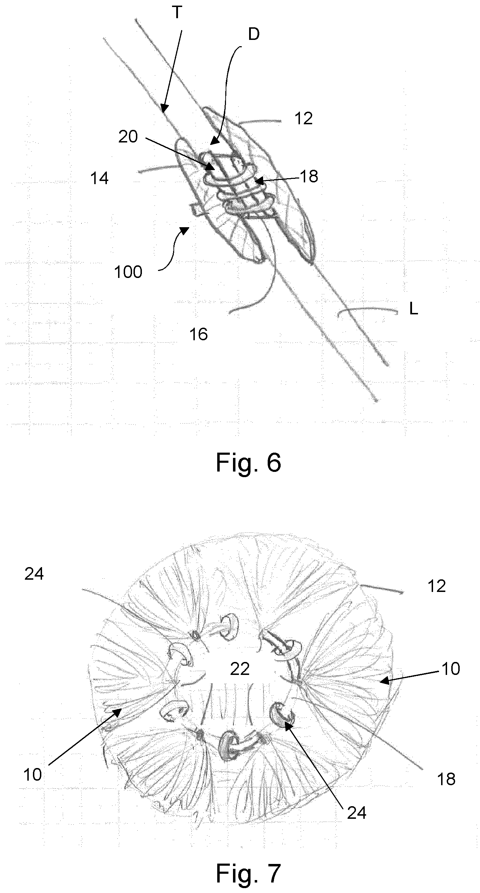

**Fig. 6** shows the medical device 100, here an occluder device 100, in an implanted state after deployment.

Here, the two expanded diameter portions 12, 14 are spaced apart from one another by the waist portion 16.

In the present embodiment, the two expanded diameter portions 12, 14 comprise a first expanded diameter portion 12, oriented toward the left side after implantation, and a second expanded diameter portion 14, oriented toward the right side after implantation (**Fig. 6**).

By the provision of a medical device 100, here an occluder device 100, so configured, the self-centering properties of the device 100 can be preserved, while the two expanded diameter portions 12, 14 are independent from the waist portion 16.

Accordingly, the medical device 100 can be more easily deployed and maintained in place after implantation, preventing the occurrence of tissue erosion and/or device prolapse.

In the present embodiment, the waist portion 16 is configured and arranged such that the expanded diameter portions 12, 14 are semi-independent in their movement to each other.

In particular, the semi-independent movement of the expanded diameter portions 12, 14, especially during endothelialization (occurring in the first months after implantation), allows further preventing the risk of tissue trauma.

In the present embodiment, the waist portion 16 is configured and arranged to at least partially control the movement of the expandable diameter portions 12, 14.

Also, in the present embodiment, the waist portion 16 is configured and arranged to connect the expandable diameter portions 12, 14 to one another, as shown in **Fig. 4****.**

Also, in the present embodiment, the waist portion 16 is configured and arranged to be placed in a cardiac defect D or hole, preferably a septal defect D or hole (**Fig. 6**).

In particular, the septal defect D or hole may be generated by a congenital heart disease such as ASD, VSD, PFO and/or PDA.

In the present embodiment, the waist portion 16 is further configured and arranged to be at least partially self-centering in said cardiac defect D or hole.

In particular, partial self-centering of the waist portion 16 is convenient in case the medical device 100 is used for the treatment of muscular VSD (MVSD).

Otherwise, the waist portion 16 may be configured and arranged to be completely self-centering.

In the present embodiment, the two expanded diameter portions 12, 14 are configured and arranged to be slidable relatively to each other, controlled in their relative movement by the waist portion 16.

In the present embodiment, the two expanded diameter portions 12, 14 are configured and arranged to be at least partly rotatable relatively to each other, controlled in their relative movement by the waist portion 16.

In the present embodiment, the two expanded diameter portions 12, 14 are configured and arranged to adjust their distance relatively to each other, controlled in their relative movement by the waist portion 16.

Conveniently, the braided strands 10 forming the fabric are made of metal, preferably Nitinol or Steel.

Alternatively, the braided strands 10 forming the fabric may be formed of a polymeric material, preferably Polyester.

In the latter case, the polymeric material, e.g. Polyester, used for the braided strands 10 may further include a metal covering, preferably comprising Nitinol or Steel.

### Summarizing the following can be said:

The present disclosure provides a medical device 100, preferably an occlusion device 100, and a method for occluding an opening in a body tissue with the occlusion device 100.

The occlusion device 100 as shown in **Fig. 3** and **Fig. 4** is specifically, though not solely, meant to occlude unwanted openings in a heart wall T.

The present disclosure is directed to an occluder device 100 comprising two aligned discs 12, 14 linked together at a waist portion 16.

The waist portion 16 is constructed such that the discs 12, 14 can act independently of each other during deployment, attachment to a body wall and during the first several months while the body tissue continues to cover it.

The discs 12, 14 can self-adjust to the surrounding heart wall T causing minimal or no stretch or sawing effect.

Each disc 12, 14 includes a waist element 18, 20 constructed of wires from the respective discs 12, 14 to make the elements 18, 20 a part of each disc 12, 14 at attachment points 22.

The elements 18, 20 may directly connect with each other or through separate circular connection elements 24, e.g. rings, on the waist elements 18, 20 of each disc 12, 14. If connection elements 24 are utilized, the connection elements 24 are loosely attached to the waist elements 18, 20.

The present disclosure has several advantages when compared to previously available devices.

The occluder device 100 will be able to address closure of ASD, PFO, all types of VSDs and PDA without inherent risk of erosion.

Further, the device 100 will ease the effective delivery of the device and reduce chances of disc prolapse.

The present disclosure focuses mainly on the waist portion 16 of the device 100 and is modified in such a way that the self-centering properties of the device 100 are preserved while the device discs 12, 14 are independent of the device waist portion 16.

Other advantages include the following:
- ASD: (Adaptable, Rotate-able, slide-able, and dis-locatable)
- Partially flexible and independent discs,
- Partially slide-able discs,
- Discs modified to prevent atrial wall trauma for atrial devices,
- Dislocate-able discs, and
- Potentially double layered Dacron mesh waist to ensure effective seal and near complete closure of the defect.

The present disclosure provides the following advantages over other devices:
1. Partial detachability so that the distal or left sided disc and proximal (right disc) can easily conform to the septal angle and surrounding tissue,
2. Partial rotatability that will prevent sawing effect of the device edge on the surrounding heart muscle and prevent tissue wall trauma,
3. The membranous VSD device design mimics surgical closure without excessive radial force to the surrounding vulnerable structures,
4. Independent disc partial movement to adjust according to surrounding structures,
5. Double layer disc for stability and assurance of complete closure, and
6. PFO device connecting waist designed to fit in lunar (slit-shaped) shaped defects

### Basic Design:

The basic design is like other available devices in that it has a double-disc design with a connecting waist. It has a nitinol-based wiring system, is self-centering, retrievable, repositionable and removable.

Referring to **Fig. 3** and **Fig. 4**, the occluder device 100 is illustrated showing two aligned discs 12, 14, linked together at the waist portion 16 (**Fig. 4**).

In the relaxed position, as illustrated in **Fig. 3**, the two discs 12, 14 overlap each other. As illustrated, the distal disc 12 has a larger diameter than the proximal disc 14 (**Figs**. **3-4**).

However, the device 100 may or may not have a disc diameter difference.

Currently available devices are made of Nitinol wires that start at a hub, make a disc, continue to the waist portion of the device.

The waist diameter is generally equal to the size of the defect D for which the device 100 is made, usually in the range from 4-40 mm.

### The Waist portion:

Advantageously, the waist portion 16 of device 100 is constructed such that the discs 12, 14 can act independently during the first several months while the body tissue continues to cover it, which is a time of rapid endothelialization.

This will help to obviate risk of tissue trauma. The discs 12, 14 can self-adjust to the surrounding heart wall T, as illustrated in **Fig. 6**, causing minimal or no stretch or sawing effect.

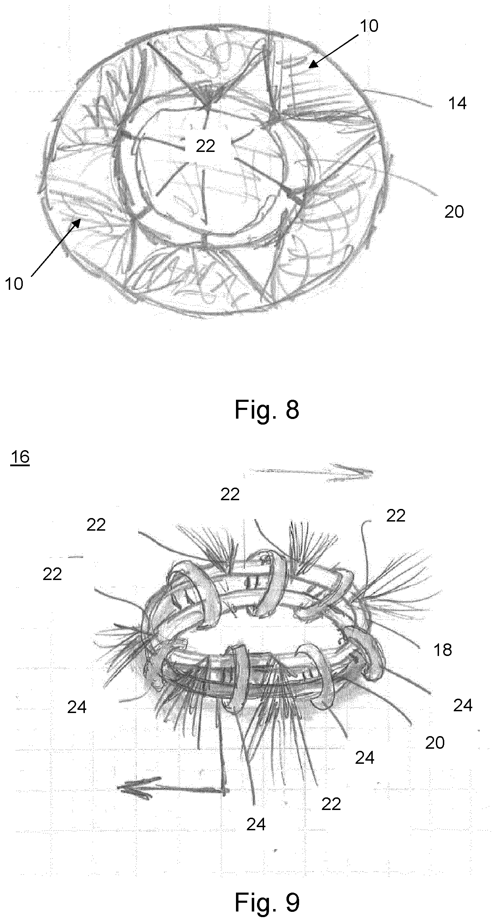

As illustrated in **Fig. 7** and **Fig. 8**, showing the ventral or underside of each disc 12, 14, each disc 12, 14 includes a waist element 18, 20 which will be made of the wires from the respective discs 12, 14.

As illustrated, the waist elements 18, 20 will be part of each disc 12, 14 at attachment points 22 on the elements 18, 20.

These converging wires will maintain the self-centering nature of the occluding device 100. These elements 18, 20 may directly connect with each other or through separate connection elements 24 on the waist elements 18, 20 of each disc 12, 14.

As illustrated in **Fig. 7**, the attachment rings 24 are only currently loosely attached to waist element 18 of disc 12. The number of attachment rings 24 per occluder device 100 will vary based upon the diameter of the waist elements 18, 20, i.e., a larger diameter means more connection elements 24 and vice versa.

These connection elements 24 will be modified to make a PFO device (oval rings so the discs are more slidable in a tunnel type PFO).

For MVSD, connection elements 24 will be made in such a way that the disc separation is around 6-9 mm. This is best illustrated in **Fig. 6****.**

The resulting discs 12, 14 will be parallel, independent, rotatable, slidable and partially dis-locatable.

**Fig. 5** illustrates the mode of insertion of the occluder device 100 using a catheter 200. As illustrated in **Fig. 6**, the main idea of the device 100 is complete modification of the connecting waist portion 16 so that it can maintain its self-centering properties while becoming easier to deploy, thereby causing less friction and trauma to the body wall. The waist portion 16 of the present invention can be modified to fit and close different types of defects as specified above.

### Alternative Embodiments:

PFO: As above and the waist portion 16 between discs 12, 14 modified to conform to the PFO anatomy.

PMVSD ("Mem. VSD" - membranous ventricular septal defect): Lunar shaped connecting waist portion16. Discs eccentric with more coverage of the ventricular septum; the right sided disc 14 to mimic surgical closure and for stability.

MVSD ("muscular VSD"): Discs 12, 14 as ASD device, but partially self-centering. PDA ("patent ductus arteriosus"): Connecting waist portion 16 like ASD, length of the waist portion 16 between 3-10 mm.

Preemie PDA: Central core wire with two attachments.

Delivery mechanism for VSDs: Like currently available devices.

For mem. VSD device, the right sided disc 14 will be modified so that it covers also all the defect and the left disc will only act as anchor.

For PDA device, the right disc 14 will be 1-3 mm larger than the waist portion 16 and the dimensions will be based upon the size of the device 100.

The length of waist portion 16 of the device 100 will essentially vary based upon the size of the device 100.

The left disc 12 will be mildly concave to conform to the inner aortic wall.

Detailed views of the first and second expanded diameter portions 12, 14 are respectively provided in **Figs. 7-8**, showing said expanded diameter portions 12, 14 from below.

The two expanded diameter portions 12, 14 may have a double-layered structure.

**Fig. 9** shows a detail of the waist portion 16 of the medical device 100, here an occluder device 100.

In particular, in the present embodiment, each of the two expanded diameter portions 12, 14 includes a respective waist element 18, 20 (**Figs. 7-8**).

The waist elements 18, 20 form the waist portion 16 when joined to one another, as shown in **Fig. 9****.**

In the present embodiment, the waist elements 18, 20 are formed of the same braided strands 10 forming the expanded diameter portions 12, 14.

Each of the waist elements 18, 20 is connected to its respective expanded diameter portion 12, 14 at one or more connection points 22, as shown in **Fig. 7-9****.**

One or more connection elements 24 are provided to connect the waist elements 18, 20 of the expanded diameter portions 12, 14 to one another, to form the waist portion 16 (**Fig. 9**).

In the shown embodiment, a plurality of connection elements 24 is provided to connect the waist elements 18, 20 to one another (**Figs. 4** and **9**).

In particular, as shown in **Fig. 7**, the connection elements 24 are provided on the waist element 18 of the first expanded diameter portion 12.

The connection elements 24 may be loosely attached to the waist portion 18 of the first expanded diameter portion 12, and thereby arranged to be rotatable.

Not shown is that further arrangements for the connection elements 24 are also possible, provided that they are suitable for their purpose.

The overall number of connection elements 24 for each medical device 100 depends upon the size, e.g. the diameter, of the waist elements 18, 20.

In particular, waist elements 18, 20 having a larger size require a larger number of connection elements 24 to ensure a reliable connection.

In a possible configuration, the one or more connection elements 24 are arranged such that the two expanded diameter portions 12, 14 are spaced apart by a distance ranging from 6 mm to 9 mm.

This configuration is particular convenient when the medical device 100, here an occluder device 100, is used for the treatment of cardiac defects D or holes generated by MVSD.

In the shown embodiment, the connection elements 24 are substantially circular in cross-section (**Figs. 4****,** **7** and **9**).

However, different shapes for the connection elements 24 are also possible according to the invention.

For instance, not shown is that the connection elements 24 may be partially rectangular, oval, triangular, or quadratic in cross-section.

Preferably, the connection elements 24 have rounded edges.

This prevents the risk of injuries to a surrounding tissue T during and/or after deployment.

Not shown is that, alternatively, the connection elements 24 can be string-shaped.

Also not shown is that, alternatively, the connection elements 24 can be substantially in the shape of an elongated "8".

In the present embodiment, the first expanded diameter portion 12 has a larger diameter than the second expanded diameter portion 14 (**Figs**. **3-4** and **6**).

Not shown is that the two expanded diameter portions 12, 14 may as well be configured to have the same diameter.

The second expanded diameter portion 14 may be 1 mm to 3 mm larger than the waist portion 16.

In principle, it is preferable that the waist portion 16 has a dimension that is generally equal to the size of the cardiac defect D or hole to be treated.

In the shown configuration, the waist portion 16 has a circular shape (**Fig. 9**), preferably with a diameter ranging between 4 mm and 40 mm.

Not shown is that, alternatively, the waist portion may have an oval shape.

Also not shown is that, alternatively, the waist portion may have a substantially lunar shape.

The latter configuration may be convenient, e.g., when the medical device 100 is used for the treatment of a cardiac defect D or hole generated by MVSD.

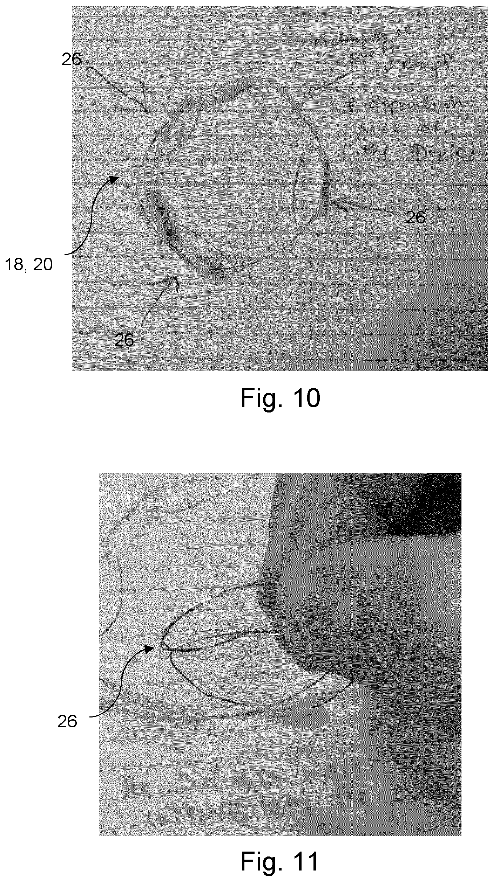

**Fig. 10** shows a detail of one of the waist portion elements 18, 20 forming the waist portion 16.

Here, the waist portion element 18, 20 includes a plurality of wire loops 26.

The wire loops 26 can be rings.

One of said wire loops 26 is illustrated in greater detail in **Fig. 11****.**

In the shown configuration, the wire loops 26 are substantially oval.

Not shown is that, alternatively, different shapes for the wire loops, e.g. substantially rectangular, are also possible.

In the present embodiment, the waist portion 16 comprises a mesh, preferably a double-layered mesh, made of a polymeric material, e.g. Polyester.

The waist portion16 may have a variable length.

In particular, the waist portion 16 may have an overall length ranging from 3 mm to 40 mm
In a possible configuration of the medical device 100 of the invention, the second expanded diameter portion 14 is configured and arranged to entirely cover a cardiac defect D or hole, preferably a septal defect D or hole, while the first expanded diameter portion 12 is configured and arranged to act as an anchor element, securing the medical device 100 to a surrounding tissue T after implantation.

Also, the first expanded diameter portion 12 may have a concave shape, preferably a mildly-concave shape, allowing for better adaption to the shape of a surrounding body cavity after implantation.

For instance, said body cavity can be the inner aortic wall.

As mentioned, the collapsible medical device 100 of the invention is preferably an occluder device 100.

The characteristics of the device 100 render it is suitable for the effective treatment of cardiac defects D or holes of different shapes and kinds, e.g. caused by congenital heart diseases such as ADS, VSD, PFO and/or PDA, without inherent risk of tissue erosion and/or device prolapse.

Any version of any component or method step of the present disclosure may be used with any other component or method step of the disclosure.

The elements described herein can be used in any combination whether explicitly described or not.

All combinations of method steps as used herein can be performed in any order, unless otherwise specified or clearly implied to the contrary by the context in which the referenced combination is made.

As used herein, the singular forms "a," "an," and "the" include plural referents unless the content clearly dictates otherwise.

Numerical ranges as used herein are intended to include every number and subset of numbers contained within that range, whether specifically disclosed or not. Further, these numerical ranges should be construed as providing support for a claim directed to any number or subset of numbers in that range. For example, a disclosure of from 1 to 10 should be construed as supporting a range of from 2 to 8, from 3 to 7, from 5 to 6, from 1 to 9, from 3.6 to 4.6, from 3.5 to 9.9, and so forth.

All patents, patent publications, and peer-reviewed publications (i.e., "references") cited herein are expressly incorporated by reference in their entirety to the same extent as if each individual reference were specifically and individually indicated as being incorporated by reference. In case of conflict between the present disclosure and the incorporated references, the present disclosure controls.

The devices, methods, compounds and compositions of the present invention can comprise, consist of, or consist essentially of the essential elements and limitations described herein, as well as any additional or optional steps, ingredients, components, or limitations described herein or otherwise useful in the art.

While this invention may be embodied in many forms, what is described in detail herein is a specific preferred embodiment of the invention. The present disclosure is an exemplification of the principles of the invention is not intended to limit the invention to the particular embodiments illustrated. It is to be understood that this invention is not limited to the particular examples, process steps, and materials disclosed herein as such process steps and materials may vary somewhat. It is also understood that the terminology used herein is used for the purpose of describing particular embodiments only and is not intended to be limiting since the scope of the present invention will be limited to only the appended claims and equivalents thereof.

In connection with the foregoing disclosure, the following aspects of the present disclosure are explicitly disclosed:
Aspect 1: A collapsible medical device (100) comprising a fabric formed of braided strands (10), the device (100) having a collapsed configuration for delivery through at least a lumen (L) in a subject, preferably a human patient, and has a generally dumbbell-shaped expanded configuration with two expanded diameter portions (12, 14) spaced apart by a waist portion (16) formed between the two expanded diameter portions (12, 14).
Aspect 2: The medical device (100) according to aspect 1, wherein the waist portion (16) is configured and arranged such that the two expanded diameter portions (12, 14) are semi-independent in their movement to each other.
Aspect 3. The medical device (100) according to aspect 1 or 2, wherein the waist portion (16) is configured and arranged to at least partially control the movement of the two expandable diameter portions (12, 14).
Aspect 4: The medical device (100) according to any one of the preceding aspects, wherein the waist portion (16) is configured and arranged to connect the two expandable diameter portions (12, 14) to one another.
Aspect 5: The medical device (100) according to any one of the preceding aspects, wherein the waist portion (16) is configured and arranged to be placed in a cardiac defect (D) or hole, preferably a septal defect or hole, in particular a septal defect (D) or hole caused by a congenital heart disease such as atrial septal defects (ASD), ventricular septal defects (VSD), patent foramen ovale (PFO), and/or patent ductus arteriosus (PDA),
   preferably wherein said waist portion (16) is further configured and arranged to be at least partially self-centering in said cardiac defect (D) or hole.
Aspect 6: The medical device (100) according to any one of the preceding aspects, wherein the two expanded diameter portions (12, 14) are configured and arranged to be slidable relatively to each other, controlled in their relative movement by the waist portion (16).
Aspect 7: The medical device (100) according to any one of the preceding aspects, wherein the two expanded diameter portions (12, 14) are configured and arranged to be at least partly rotatable relatively to each other, controlled in their relative movement by the waist portion (16).
Aspect 8: The medical device (100) according to any one of the preceding aspects, wherein the two expanded diameter portions (12, 14) are configured and arranged to adjust their distance relatively to each other, controlled in their relative movement by the waist portion (16).
Aspect 9: The medical device (100) according to any one of the preceding aspects, wherein said braided strands (10) forming the fabric are made of metal, preferably wherein said metal is Nitinol or Steel, or
   said braided strands (10) forming the fabric are made of a polymeric material, preferably wherein said polymeric material is Polyester, optionally wherein said polymeric material has metal covering, preferably wherein said metal covering comprises Nitinol or Steel.
Aspect 10: The medical device (100) according to any one of the preceding aspects, wherein each of the two expanded diameter portions (12, 14) includes a respective waist element (18, 20), made of the same braided strands (10) forming the expanded diameter portions (12, 14), said waist elements (18, 20) forming the waist portion (16) when joined to one another,
   wherein each of the waist elements (18, 20) is connected to a respective expanded diameter portion (12, 14) at one or more connection points (22).
Aspect 11: The medical device (100) according to aspect 10, wherein one or more connection elements (24) are provided to join the waist elements (18, 20) of the expanded diameter portions (12, 14) to one another to form the waist portion (16),
   preferably wherein said one or more connection elements (24) are provided on the waist element (18) of the first expanded diameter portion (12).
Aspect 12: The medical device (100) according to aspect 11, wherein the one or more connection elements (24) are arranged such that the two expanded diameter portions (12, 14) are spaced apart by a distance ranging from 6 mm to 9 mm.
Aspect 13: The medical device (100) according to aspects 11 or 12, wherein the one or more connection elements (24) are circular, partially rectangular, oval, triangular, or quadratic in cross-section, preferably wherein said one or more connection elements (24) have rounded edges to facilitate implantation and/or prevent the risk of injuries to a surrounding tissue (T) during and/or after implantation, or
   wherein said one or more connection elements (24) are string-shaped, or wherein said one or more connection elements (24) are substantially in the shape of an elongated "8".
Aspect 14: The medical device (100) according to any one of the preceding aspects, wherein the first expanded diameter portion (12) has a larger diameter than the second expanded diameter portion (14).
Aspect 15: The medical device (100) according to any one of aspects 1 to 13, wherein the two expanded diameter portions (12, 14) have the same diameter.
Aspect 16: The medical device (100) according to any one of the preceding aspects, wherein at least the second expanded diameter portion (14) is 1 mm to 3 mm larger than the waist portion (16).
Aspect 17: The medical device (100) according to any one of the preceding aspects, wherein the second expanded diameter portion (14) is configured and arranged to entirely cover a cardiac defect (D) or hole, preferably a septal defect or hole, and the first expanded diameter portion (12) is configured and arranged to act as an anchor element, securing the medical device (100) to a surrounding tissue (T) of the lumen (L) after implantation.
Aspect 18: The medical device (100) according to any one of the preceding aspects, wherein the first expanded diameter portion (12) has a concave shape for better adaption to the shape of a surrounding body cavity after implantation, preferably wherein said body cavity is the inner aortic wall.
Aspect 19: The medical device (100) according to any one of the preceding aspects, wherein the two expanded diameter portions (12, 14) have a double-layered structure.
Aspect 20: The medical device (100) according to any one of the preceding aspects, wherein the waist portion (16) has a circular shape, preferably with a diameter ranging between 4 mm and 40 mm.
Aspect 21: The medical device (100) according to any one of aspects 1 to 19, wherein the waist portion (16) has an oval shape.
Aspect 22: The medical device (100) according to any one of aspects 1 to 19, wherein the waist portion (16) has a substantially lunar shape.
Aspect 23: The medical device (100) according to any one of the preceding aspects, wherein the waist portion (16) comprises a mesh, preferably a double-layered mesh, made of a polymeric material,
   preferably wherein said polymeric material is Polyester.
Aspect 24: The medical device (100) according to any one of the preceding aspects, wherein the waist portion (16) has a variable length.
Aspect 25: The medical device (100) according to any one of the preceding aspects, wherein the waist portion (16) has an overall length ranging from 3 mm to 40 mm.
Aspect 26: The medical device (100) according to any one of the preceding aspects, wherein the medical device (100) is an occluder device, in particular adapted for use in a subject, preferably a human patient, affected by a congenital heart disease such as atrial septal defects (ASD), ventricular septal defects (VSD), patent foramen ovale (PFO), and/or patent ductus arteriosus (PDA).

### Reference list

- 100: Collapsible medical device (occluder device)

- 10: Braided strands
- 12: First expanded diameter portion
- 14: Second expanded diameter portion
- 16: Waist portion
- 18: Waist element (first expanded diameter portion)
- 20: Waist element (second expanded diameter portion)
- 22: Connection point
- 24: Connection element
- 26: Wire loops

- 200: Catheter

- L: Lumen
- D: Cardiac defect
- T: Cardiac tissue, heart wall

## Claims

1. A collapsible medical device (100) comprising a fabric formed of braided strands (10), the device (100) having a collapsed configuration for delivery through at least a lumen (L) in a subject, preferably a human patient, and has a generally dumbbell-shaped expanded configuration with two expanded diameter portions (12, 14) spaced apart by a waist portion (16) formed between the two expanded diameter portions (12, 14).

2. The medical device (100) according to claim 1,
**characterized in that**
the waist portion (16) is configured and arranged such that the two expanded diameter portions (12, 14) are semi-independent in their movement to each other.

3. The medical device (100) according to claim 1 or 2,
**characterized in that**
the waist portion (16) is configured and arranged to at least partially control the movement of the two expandable diameter portions (12, 14).

4. The medical device (100) according to any one of the preceding claims,
**characterized in that**
the waist portion (16) is configured and arranged to connect the two expandable diameter portions (12, 14) to one another.

5. The medical device (100) according to any one of the preceding claims,
**characterized in that**
the waist portion (16) is configured and arranged to be placed in a cardiac defect (D) or hole, preferably a septal defect or hole, in particular a septal defect (D) or hole caused by a congenital heart disease such as atrial septal defects (ASD), ventricular septal defects (VSD), patent foramen ovale (PFO), and/or patent ductus arteriosus (PDA),
preferably wherein said waist portion (16) is further configured and arranged to be at least partially self-centering in said cardiac defect (D) or hole.

6. The medical device (100) according to any one of the preceding claims,
**characterized in that**
the two expanded diameter portions (12, 14) are configured and arranged to be slidable relatively to each other, controlled in their relative movement by the waist portion (16).

7. The medical device (100) according to any one of the preceding claims,
**characterized in that**
the two expanded diameter portions (12, 14) are configured and arranged to be at least partly rotatable relatively to each other, controlled in their relative movement by the waist portion (16).

8. The medical device (100) according to any one of the preceding claims,
**characterized in that**
the two expanded diameter portions (12, 14) are configured and arranged to adjust their distance relatively to each other, controlled in their relative movement by the waist portion (16).

9. The medical device (100) according to any one of the preceding claims,
**characterized in that**
said braided strands (10) forming the fabric are made of metal, preferably wherein said metal is Nitinol or Steel, or
said braided strands (10) forming the fabric are made of a polymeric material, preferably wherein said polymeric material is Polyester, optionally wherein said polymeric material has metal covering, preferably wherein said metal covering comprises Nitinol or Steel.

10. The medical device (100) according to any one of the preceding claims,
**characterized in that**
each of the two expanded diameter portions (12, 14) includes a respective waist element (18, 20), made of the same braided strands (10) forming the expanded diameter portions (12, 14), said waist elements (18, 20) forming the waist portion (16) when joined to one another,
wherein each of the waist elements (18, 20) is connected to a respective expanded diameter portion (12, 14) at one or more connection points (22).

11. The medical device (100) according to claim 10,
**characterized in that**
one or more connection elements (24) are provided to join the waist elements (18, 20) of the expanded diameter portions (12, 14) to one another to form the waist portion (16),
preferably wherein said one or more connection elements (24) are provided on the waist element (18) of the first expanded diameter portion (12).

12. The medical device (100) according to claim 11,
**characterized in that**
the one or more connection elements (24) are arranged such that the two expanded diameter portions (12, 14) are spaced apart by a distance ranging from 6 mm to 9 mm.

13. The medical device (100) according to claims 11 or 12,
**characterized in that**
the one or more connection elements (24) are circular, partially rectangular, oval, triangular, or quadratic in cross-section, preferably wherein said one or more connection elements (24) have rounded edges to facilitate implantation and/or prevent the risk of injuries to a surrounding tissue (T) during and/or after implantation, or
wherein said one or more connection elements (24) are string-shaped, or wherein said one or more connection elements (24) are substantially in the shape of an elongated "8".

14. The medical device (100) according to any one of the preceding claims,
**characterized in that**
the first expanded diameter portion (12) has a larger diameter than the second expanded diameter portion (14).

15. The medical device (100) according to any one of claims 1 to 13,
**characterized in that**
the two expanded diameter portions (12, 14) have the same diameter.
